Europäisches Patentamt

European Patent Office

Office européen des brevets

(11) Veröffentlichungsnummer: **0 322 352 B1**

(19)

(12) **EUROPÄISCHE PATENTSCHRIFT**

(45) Veröffentlichungstag der Patentschrift: **15.12.93**

(51) Int. Cl.⁵: **A61K 31/575**, A61K 31/565

(21) Anmeldenummer: **88730281.8**

(22) Anmeldetag: **20.12.88**

(54) Verwendung von Antigestagenen zur Herstellung von Arzneimitteln.

(30) Priorität: **21.12.87 DE 3744054**

(43) Veröffentlichungstag der Anmeldung:
**28.06.89 Patentblatt 89/26**

(45) Bekanntmachung des Hinweises auf die Patenterteilung:
**15.12.93 Patentblatt 93/50**

(84) Benannte Vertragsstaaten:
**AT BE CH DE ES FR GB GR IT LI LU NL SE**

(56) Entgegenhaltungen:

**NEW ENGLAND JOURNAL OF MEDICINE, Band 315, Nr. 25, 18. Dezember 1986, Seiten 1565-1570; Massachusetts Med. Soc., US B. COUZINET et al.: "Termination of early pregnancy by the progesterone antagonist RU 486 (Mifepristone)"**

**HUMAN REPRODUCTION, Band 1, Nr. 2, Februar 1986, Seiten 107-110 IRL PRESS LTD, Oxford, GB; E.E. BAULIEU et al.: "Antiprogesterone activity of RU 486 and its contragestive and other applications"**

(73) Patentinhaber: **SCHERING AKTIENGESELL-SCHAFT Berlin und Bergkamen**
**Müllerstrasse 170/178**
**Postfach 65 03 11**
**D-13303 Berlin(DE)**

(72) Erfinder: **Chwalisz, Krzysztof, Dr.**
**Luzerner Strasse 1d**
**D-1000 Berlin 45(DE)**
Erfinder: **Beier, Sybille, Dr.**
**Uhlandstrasse 121**
**D-1000 Berlin 31(DE)**
Erfinder: **Esch, Angelika**
**Birlingerweg 15**
**D-1000 Berlin 22(DE)**
Erfinder: **Elger, Walter, Dr.**
**Schorlemer Allee 12B.**
**D-1000 Berlin 33(DE)**

AM. J. OBSTET. GYNECOL., Band 157, Nr. 6, Dezember 1987, Seiten 1487-1495; G.J. HALUSKA et al.: "Temporal changes in uterine activity and prostaglandin response to RU486 in rhesus macaques in late gestation"

J. GYNECOL. OBSTET. BIOL. REPROD. Band 16, Nr. 2, 1987, Seiten 247-249; H. FERNANDEZ et al.: "Intérêt du RU 486 dans les échecs d'IVG"

ACTA ENDOCRINOL., Suppl. 283, 1987, Seiten 113-114, Copenhagen, DK; K. SCHWALISZ et al.: "The effect of antigestagen ZK 98.299 on the uterine cervix"

ZENT. BL. GYNAKOL., Band 110, Nr. 12, 1988, Seiten 766-771 G. GORETZLEHNER et al.: "Antigestagene"

CONTRACEPTION, Band 38, Nr. 3, 1988, Seiten 301-312 A. RADESTAD et al.: "Induced cervical ripening with mifepristone in first trimester abortion"

HUMAN REPRODUCTION, Band 3, Nr. 5, Juli 1988; Seiten 583-584; F. DURLOT et al.: "Efficacy of progesterone antagonist RU 486 (Mifepristone) for pre-operative cervical dilatation during first trimester abortion"

BAILLIERE'S CLINICAL OBSTETRICS AND CYNAECOLOGY, Band 2, Nr. 3, September 1988, Seiten 631-638 A. ULMANN et al.: "Anti-progesterones in obstetrics, ectopic pregnancies and gynaecological malignancy"

Dilatation of the Uterine Cervix, F.Naftolin and P.G. Stubblefield (editors) Raven Press, New York; Koob T. et al. (1980)

Dilatation of the Uterine Cervix, F. Naftolin and P.G. Stubblefield (editors) Raven Press, New York; Kirton, K.T. (1980)

J. Maternal-fetal Med. 1: 213-223, Leppert P. (1992)

Lancet 335/8700. 1238-1240, Gupta JK et al. (1990)

Hum. Reprod. 7: 601-605, van der Schoot P. (1992)

JAMA, Oct. 6, 1989, vol. 262, no. 13, p. 1808-1814, E.-E. Baulieu

Lancet 1990, 335, 1238-1240, Gupta, JK et al.

**Beschreibung**

Die Erfindung betrifft die Verwendung von Antigestagenen zur Herstellung von Arzneimitteln mit zervixerweichendem Effekt außerhalb der Gravidität.

Es ist bekannt, daß Antigestagene, wie zum Beispiel 11$\beta$-[4-N,N-Dimethylamino)-phenyl]-17$\alpha$-hydroxy-17$\beta$-(3-hydroxypropyl)-13$\alpha$-methyl-4,9(10)-gonadien-3-on, während der Gravidität einen Einfluß auf die Zervix haben. Bei nichtgraviden und am Anfang der Gravidität stehenden Tieren ist die Zervix sehr hart. In der zweiten Hälfte der Gravidität vollzieht sich eine progressive Erweichung und Erweiterung der Zervix und erreicht ein Maximum kurz vor der Geburt. Die Zervixerweichung und -erweiterung kann mit Antigestagenen während der Gravidität induziert werden (Acta Endocrinol. (Copenhagen) Suppl. 283 (1987) 113].

Es wurde nun gefunden, daß der zervixerweichende Effekt von Antigestagenen nicht auf die gravide Zervix beschränkt ist.

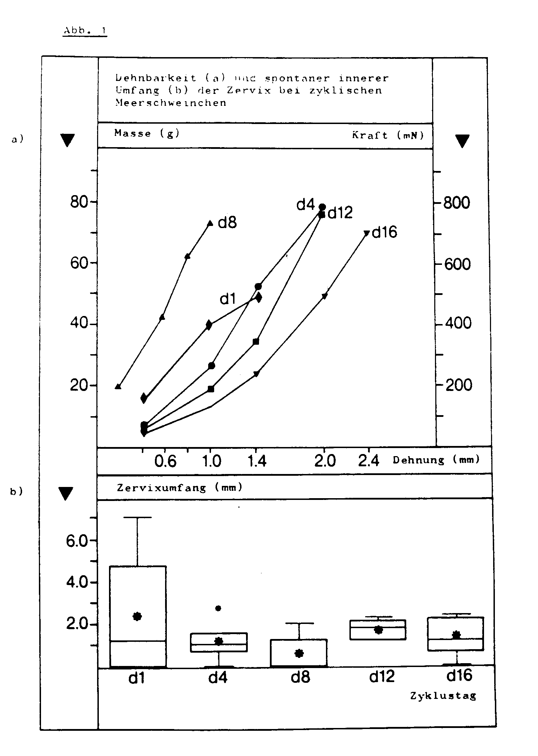

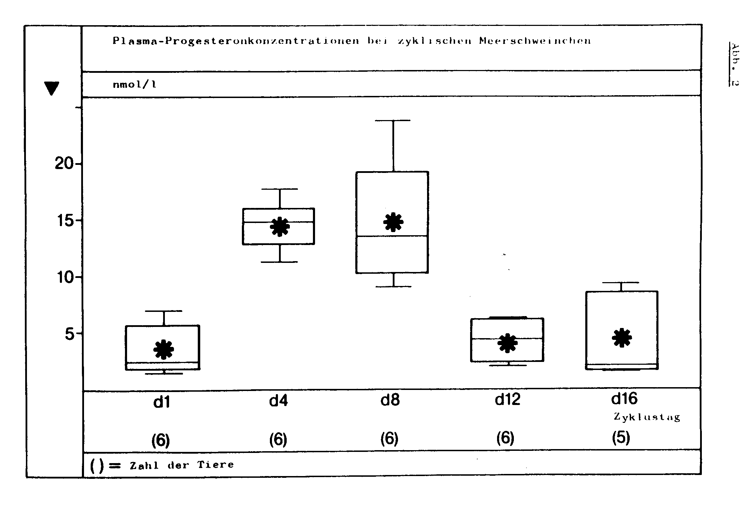

Bei nicht-graviden Tieren stehen die zyklischen Veränderungen der Dehnbarkeit der Zervix im umgekehrten Verhältnis zum Serum-Progesteron-Spiegel (s. Abb. 1 und 2). Bei Meerschweinchen findet man am 8. Zyklustag, wenn die Progesteronspiegel am höchsten sind, eine sehr harte Zervix. Nach Behandlung mit Antigestagenen wird überraschenderweise eine Zervixerweichung beobachtet, obwohl die Serum-Progesteron-Spiegel unter der Behandlung ansteigen (s. Abb. 3 und 4).

Die erfindungsgemäße Antigestagenbehandlung kann auch in der Humanmedizin bei nichtgraviden Frauen Anwendung finden, insbesondere als Vorbehandlung vor verschiedenen operativen Eingriffen am Uterus, wie beispielsweise Hysteroskopie, Einlegen von IUD oder Kürettage. Durch die erfindungsgemäße Behandlung werden Schmerzen und Verletzungen an der Zervix weitgehend vermieden.

Je nach dem gewünschten Effekt kann die Behandlung einmal oder mehrere Male erfolgen, in der Regel wird die Behandlung täglich einmal an 1 bis 4 Tagen vor dem Eingriff vorgenommen.

Die Antigestagene werden gemäß vorliegender Erfindung in Mengen von im allgemeinen 2 bis 50 mg, vorzugsweise 5 bis 20 mg, 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on pro Tag oder einer biologisch äquivalenten Menge eines anderen Antigestagens eingesetzt.

Als Antigestagene kommen alle Verbindungen infrage, die eine starke Affinität zum Gestagenrezeptor (Progesteronrezeptor) besitzen und dabei keine eigene gestagene Aktivität zeigen. Als kompetitive Progesteron-antagonisten kommen beispielsweise folgende Steroide infrage:

11$\beta$-[(4-N,N-Dimethylamino)-phenyl]-17$\beta$-hydroxy-17$\alpha$-propinyl-4,9(10)-estradien-3-on,

11$\beta$-[(4-N,N-Dimethylamino)-phenyl]-17$\beta$-hydroxy-18-methyl-17$\alpha$-propinyl-4,9(10)-estradien-3-on und

11$\beta$-[(4-N,N-Dimethylamino)-phenyl]-17a$\beta$-hydroxy-17a$\alpha$-propinyl-D-homo-4,9(10)-16-estratrien-3-on (Europäische Patentanmeldung 82400025.1 - Veröffentlichungsnummer 0 057 115),

11$\beta$-Methoxyphenyl-17$\beta$-hydroxy-17$\alpha$-ethinyl-4,9(10)-estradien-3-on (Steroids 37 (1981) 361-382),

11$\beta$-[(4-N,N-Dimethylamino)-phenyl]-17$\beta$-hydroxy-17$\alpha$-(hydroxyprop-1-(Z)-enyl)-4,9(10)-estradien-3-on (Europäische Patentanmeldung 847300147.0 - Veröffentlichungsnummer 0 147 361),

11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on (Europäische Patentanmeldung 84730062.1 - Veröffentlichungsnummer 0 129 499).

Die Antigestagene können zum Beispiel lokal, topisch, enteral oder parenteral appliziert werden.

Für die bevorzugte orale Applikation kommen insbesondere Tabletten, Dragees, Kapseln, Pillen, Suspensionen oder Lösungen infrage, die in üblicher Weise mit den in der Galenik gebräuchlichen Zusätzen und Trägersubstanzen hergestellt werden können. Für die lokale oder topische Anwendung kommen beispielsweise Vaginalzäpfchen, Vaginalgel oder transdermale Systeme wie Hautpflaster infrage.

Bei topischer Anwendung vorzugsweise ein Gel-Volumen von 1.0 bis 2,5 ml appliziert (Dosisbereich: 0,5 mg bis 25,0 mg des Wirkstoffs pro Applikation).

Eine Dosierungseinheit enthält etwa 2 bis 50 mg 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on oder eine biologisch äquivalente Menge eines anderen Antigestagens.

Die nachstehenden Beispiele sollen die galenische Formulierung von Antigestagenen erläutern.

**Beispiel 1**

```
Zusammensetzung einer Tablette mit 10 mg  11ß-[(4-N,N-Dimethylamino)-
phenyl]-17α-hydroxy-17ß-(3-hydroxypropyl)-13α-methyl-4,9(10)-gonadien-
3-on  zur oralen Applikation
```

```
  10,0 mg   11ß-[(4-N,N-Dimethylamino)-phenyl]-17α-hydroxy-17ß-
            (3-hydroxypropyl)-13α-methyl-4,9(10)-gonadien-3-on
 140,5 mg   Laktose
  69,5 mg   Maisstärke
   2,5 mg   Polyvinylpyrrolidon 25
   2,0 mg   Aerosil
   0,5 mg   Magnesiumstearat
 225,0 mg   Gesamtgewicht
 ========
```

**Beispiel 2**

Zusammensetzung einer öligen Lösung mit 50 mg 11$\beta$[(4-N,N-Dimethylamino)-phenyl]-17$\alpha$-hydroxy-17$\beta$-(3-hydroxypropyl)-13$\alpha$-methyl-4,9(10)-gonadien-3-on zur parenteralen Applikation

In je 1 ml Rizinusöl/Benzylbenzoat im Volumenverhältnis 6:4 werden 50 mg des Antigestagens gelöst.

**Beispiel 3**

Zusammensetzung und Herstellung von Lipogelen mit

| a) 0,05 Gew.-% Antigestagen | | b) 1 Gew.-% Antigestagen |
|---|---|---|
| 0,5 mg | 10,0 mg | 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on als Antigestagen |
| 47,0 mg | 47,0 mg | Benzylbenzoat |
| 887,5 mg | 878,0 mg | Neutralöl (Miglyol 812[(R)]) |
| 65,0 mg | 65,0 mg | Kolloidales Siliciumdioxid (Aerosil 200[(R)]) |
| 1.000,0 mg | 1.000,0 mg | Lipogel |

Zur Herstellung werden zunächst unter Rühren die flüssigen Komponenten portionsweise dem Antigestagen zugegeben und weitergerührt, bis eine homogene Suspension oder Lösung erreicht ist. In die Suspension oder Lösung wird das Siliciumdioxid als Gelbildner eingearbeitet. Nach einer Ruhezeit von ungefähr 24 Stunden wird das Gel portioniert, beispielsweise in Spritzen oder Tuben aus Kunststoff.

**Beispiel 4**

Zusammensetzung und Herstellung von Hydrogelen mit

| a) 0,05 Gew.-% Antigestagen | b) 1 Gew.-% Antigestagen | |
|---|---|---|
| 0,5 mg | 10,0 mg | Antigestagen (wie in Beispiel 3) |
| 180,0 mg | 180,0 mg | Poloxamer (Pluronic F127(R)) |
| 819,5 mg | 810,0 mg | bidestilliertes Wasser |
| 1.000,0 mg | 1.000,0 mg | Hydrogel |

Zur Herstellung des Hydrogels wird das Antigestagen in dem in kaltem Wasser gelösten Poloxamer suspendiert, gegebenenfalls erst unmittelbar vor der Anwendung. Durch die am Wirkort erhöhte Temperatur (vgl. mit Raumtemperatur) erfolgt eine Verfestigung des Hydrogels.

Sowohl die lipophilen als auch die hydrophilen Gele der Beispiele 3a, b) bzw. 4a, b) können nach den üblichen Verfahren zur Verminderung der Keimzahl auch steril hergestellt werden.

**Pharmakologische Beobachtungen**

Behandlungsgruppen:

Nichtgravide Meerschweinchen erhielten täglich vom 8. bis 11. Tag des ca. 16tägigen Zyklus (Lutealphase) 10 mg 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on (A) oder 10 mg 11$\beta$-[(4-N,N-Dimethylamino)-phenyl]-17$\beta$-hydroxy-17$\alpha$-propinyl-4,9-(10)-estradien-3-on (B).

Die Testsubstanzen wurden in 1 ml Benzylbenzoat/Rizinusöl (1:2) gelöst und subcutan injiziert.

Kontrollgruppen:

Eine Gruppe von Tieren wurde vom 8. bis 11. Tag des Zyklus mit Vehikel behandelt.

Bei der zweiten Kontrollgruppe wurde die Hysterektomie am 8. Zyklustag vorgenommen.

Durchführung der Messungen:

Die Dehnbarkeit der Zervix wurde am 12. Zyklustag mit Hilfe eines speziell konstruierten Gerätes (DE-P-37 19 380, DE-P-37 19 381) in vitro gemessen. Am gleichen Tag wurden auch die Serum-Progesteronwerte bestimmt.

Ergebnis

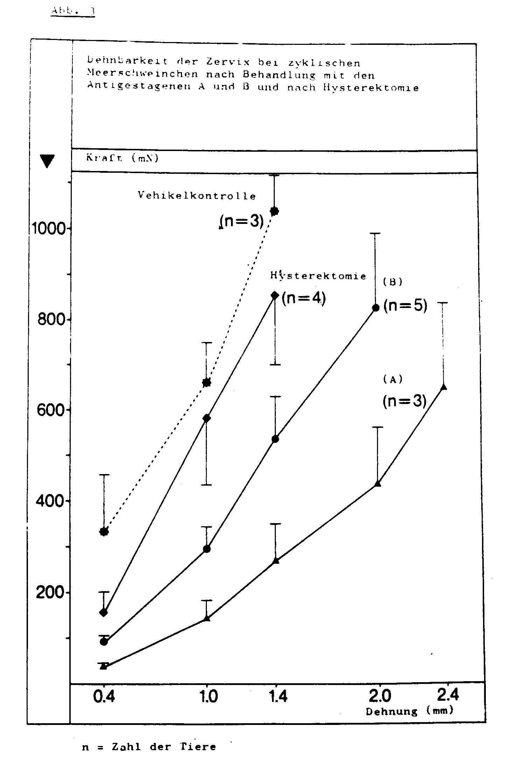

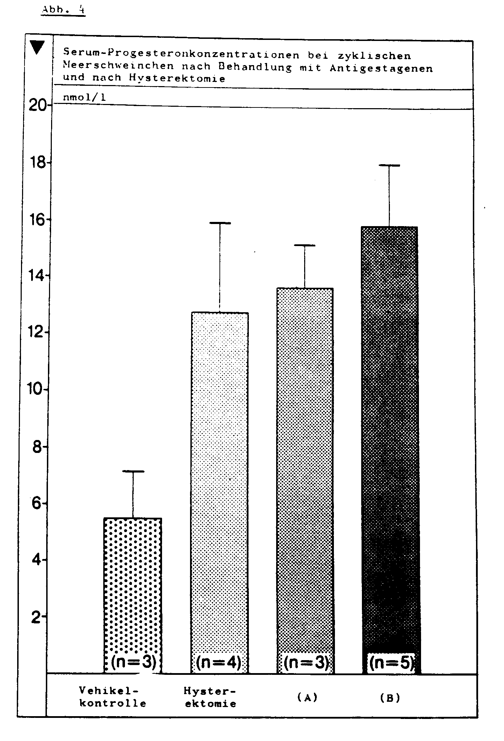

Aus den Abbildungen 3 und 4 ist ersichtlich, daß am 12. Zyklustag die Dehnbarkeit der Zervix bei den mit Antigestagen behandelten Tieren im Vergleich zu den Kontrollen stark zunimmt. Die Serum-Progesteronwerte in beiden Behandlungsgruppen A und B waren im Vergleich zur Vehikel-Kontrolle erhöht und entsprechen denjenigen der zweiten Kontrollgruppe (d.h. nach Hysterektomie). Sowohl Hysterektomie als auch Behandlung mit Antigestagenen bewirken einen antiluteolytischen Effekt, d.h. hohe Progesteronwerte am Tag 12 des Zyklus.

**Patentansprüche**

1. Verwendung von Antigestagenen zur Herstellung von Arzneimitteln zur Behandlung nichtgravider Frauen vor operativen Eingriffen am Uterus.

2. Verwendung von 11$\beta$-[(4-N,N-Dimethylamino)-phenyl]-17$\beta$-hydroxy-17$\alpha$-propinyl-4,9(10)-estradien-3-on, 11$\beta$-[(4-N,N-Dimethylamino)-phenyl]-17$\beta$-hydroxy-18-methyl-17$\alpha$-propinyl-4,9(10)-estradien-3-on, 11$\beta$-[(4-N,N-Dimethylamino)-phenyl]-17a$\beta$-hydroxy-17a$\alpha$-propinyl-D-homo-4,9(10)-16-estratrien-3-on, 11$\beta$-Methoxyphenyl-17$\beta$-hydroxy-17$\alpha$-ethinyl-4,9(10)-estradien-3-on, 11$\beta$-[(4-N,N-Dimethylamino)-phenyl]-17$\beta$-hydroxy-17$\alpha$-(hydroxyprop-1-(Z)-enyl)-4,9(10)-estradien-3-on oder

11$\beta$-[(4-N,N-Dimethylamino)-phenyl]-17$\alpha$-hydroxy-17$\beta$-(3-hydroxy-propyl)-13$\alpha$-methyl-4,9(10)-gonadien-3-on,
als Antigestagene nach Anspruch 1.

## Claims

1. Use of antigestagens for the preparation of medicaments for the treatment of non-pregnant women prior to uterine surgery.

2. Use of 11$\beta$-[(4-N,N-dimethylamino)-phenyl]-17$\beta$-hydroxy-17$\alpha$-propynyl-4,9(10)-estradien-3-one,
11$\beta$-[(4-N,N-dimethylamino)-phenyl]-17$\beta$-hydroxy-18-methyl-17$\alpha$-propynyl-4,9(10)-estradien-3-one,
11$\beta$-[(4-N,N-dimethylamino)-phenyl]-17a$\beta$-hydroxy-17a$\alpha$-propynyl-D-homo-4,9(10)-16-estratrien-3-one,
11$\beta$-methoxyphenyl-17$\beta$-hydroxy-17$\alpha$-ethynyl-4,9(10)-estradien-3-one,
11$\beta$-[(4-N,N-dimethylamino)-phenyl]-17$\beta$-hydroxy-17$\alpha$-(hydroxyprop-1-(Z)-enyl)-4,9(10)-estradien-3-one
or
11$\beta$-[(4-N,N-dimethylamino)-phenyl]-17$\alpha$-hydroxy-17$\beta$-(3-hydroxypropyl)-13$\alpha$-methyl-4,9(10)-gonadien-3-one
as antigestagens according to claim 1.

## Revendications

1. Emploi d'antiprogestatifs pour préparer des médicaments destinés au traitement de femmes qui ne sont pas enceintes avant une opération de l'utérus

2. Emploi comme antiprogestatifs selon la revendication 1 des composés suivants :
11$\beta$-[(4-N,N-diméthylamino)-phényl]-17$\beta$-hydroxy-17$\alpha$-propynyl-4,9(10)-estradién-3-one,
11$\beta$-[(4-N,N-diméthylamino)-phényl]-17$\beta$-hydroxy-18-méthyl-17$\alpha$-propynyl-4,9(10))estradién-3-one,
11$\beta$-[(4-N,N-diméthylamino)-phényl]-17a$\beta$-hydroxy-17a$\alpha$-propynyl-D-homo-4,9(10)-16-estradién-3-one,
11$\beta$-méthoxyphényl-17$\beta$-hydroxy-17$\alpha$-ethynyl-4,9(10)-estradién-3-one,
11$\beta$-[(4-N,N-diméthylamino)-phényl]-17$\beta$-hydroxy-17$\alpha$-(hydroxyprop-1-(Z)-ényl)-4,9(10)-estradién-3-one
ou
11$\beta$-[(4-N,N-diméthylamino-phényl]-17$\alpha$-hydroxy-17$\beta$-(3-hydroxy-propyl)-13$\alpha$-méthyl-4,9(10)-gonadién-3-one.

Abb. 1

Dehnbarkeit (a) und spontaner innerer Umfang (b) der Zervix bei zyklischen Meerschweinchen

Abb. 2

Plasma-Progesteronkonzentrationen bei zyklischen Meerschweinchen

Abb. 3

Dehnbarkeit der Zervix bei zyklischen
Meerschweinchen nach Behandlung mit den
Antigestagenen A und B und nach Hysterektomie

▼  Kraft (mN)

Vehikelkontrolle

(n=3)

1000

Hysterektomie    (B)
(n=4)    (n=5)

800

(A)
(n=3)

600

400

200

0.4    1.0    1.4    2.0    2.4
Dehnung (mm)

n = Zahl der Tiere

Abb. 4

Serum-Progesteronkonzentrationen bei zyklischen Meerschweinchen nach Behandlung mit Antigestagenen und nach Hysterektomie